# EUROPÄISCHE PATENTSCHRIFT

(11) **EP 0 836 471 B1**
(45) Veröffentlichungstag und Bekanntmachung des Hinweises auf die Patenterteilung: **25.10.2000**
(21) Anmeldenummer: 96923908.6
(22) Anmeldetag: 24.06.1996
(51) Int. Cl.: A61K 7/48, A61K 7/50

(54) **HAUTPFLEGEMITTEL ENTHALTEND NICHTIONISCHE TENSIDE UND KATIONISCHE BIOPOLYMERE**
SKIN-CARE AGENTS CONTAINING NONIONIC SURFACTANTS AND CATIONIC BIOPOLYMERS
AGENTS DE SOINS POUR LA PEAU CONTENANT DES TENSIOACTIFS NON IONIQUES ET DES BIOPOLYMERES CATIONIQUES

(30) Priorität: 03.07.1995 DE 19524121
(43) Veröffentlichungstag der Anmeldung: 22.04.1998
(73) Patentinhaber: Cognis Deutschland GmbH, 40589 Düsseldorf (DE)
(72) Erfinder: TANABE, Bettina, D-41352 Korschenbroich (DE); WACHTER, Rolf, D-40595 Düsseldorf (DE)
(86) Internationale Anmeldenummer: EP9602751
(87) Internationale Veröffentlichungsnummer: WO9702012

(56) Entgegenhaltungen:
- EP-A- 0 224 045
- EP-A- 0 366 070
- WO-A-94/03150
- DE-A- 4 422 404
- DATABASE WPI Week 9250 Derwent Publications Ltd., London, GB; AN 92-411555 XP002016634 "Detergent compsn. having good styling performance - contains sugar-based nonionic surfactant and water-soluble chitin" & JP,A,04 308 524 (KAO) , 30.Oktober 1992
- DATABASE WPI Week 9108 Derwent Publications Ltd., London, GB; AN 91-054729 XP002016635 "Detergent compsn. imparting smoothness and wetness to skin - comprises anionic surfactant(s) nonionic surfactants(s) and water-soluble cpds., derived from chitin or chitosan" & JP,A,03 005 414 (AJINOMOTO) , 11.Januar 1991

## Beschreibung

### Gebiet der Erfindung

Die Erfindung betrifft Hautpflegemittel mit einem Gehalt an nichtionischen Tensiden und kationischen Biopolymeren sowie die Verwendung von kationischen Biopolymeren zur Verbesserung der dermatologischen Eigenschaften nichtionischer Tenside.

### Stand der Technik

Eine wesentliche Eigenschaft von Tensidmolekülen besteht darin, nicht miteinander mischbare Phasen zu emulgieren. Aus diesem Grunde sind grenzflächenaktive Stoffe übliche Bestandteile auch in kosmetischen Zubereitungen wie beispielsweise Haut- und Haarpflegeprodukten. Die im Fall der Emulsionsbildung erwünschte Grenzflächenaktivität besitzt jedoch auf der anderen Seite den Nachteil, daß Tenide auch Hautschichten penetrieren und dort Schädigungen hervorrufen können. Aus diesem Grund besteht in der Kosmetik ein ständiges Bedürfnis nach Tensiden mit gutem Emulgiervermögen und besonderer dermatologischer Verträglichkeit.

In der Praxis finden als Emulgatoren für kosmetische Produkte überwiegend nichtionische Tenside, vorzugsweise Fettalkoholpolyglycolether und zunehmend auch Alkyloligoglucoside Anwendung, die für ihr geringes Irritationspotential und ihre gute Hautverträglichkeit bekannt sind. Gegegebenenfalls werden diese nichtionischen Emulgatoren mit Proteinabbauprodukten kombiniert, die die dermatologische Verträglichkeit von Tensiden im allgemeinen über eine Komplexierung mit den Eiweißmolekülen der Haut verbessern können [vgl. G.Schuster und A.Domsch in **Seifen-Öle-Fette-Wachse, 108, 177 (1982)]**. Trotz all dieser Ansätze besteht nach wie vor die Gefahr, daß nichtionische Emulgatoren und hier wiederum besonders Fettalkoholpolyglycolether bei besonders empfindlichen Probanden Hautschädigungen hervorrufen können.

Demzufolge hat die komplexe Aufgabe der Erfindung darin bestanden, zum einen die dermatologische Verträglichkeit von nichtionischen Tensiden zu verbessern und zum anderen Pflegemittel auf Basis nichtionischer Tenside zur Verfügung zu stellen, die bei Auftragen auf geschädigter Haut zu einer Straffung führen und demnach sogar eine Repairwirkung besitzen.

### Beschreibung der Erfindung

Gegenstand der Erfindung sind Hautpflegemittel mit Repairwirkung, enthaltend
(a) nichtionische Tenside und
(b) kationische Biopolymere.

Überraschenderweise wurde gefunden, daß schon der Zusatz sehr geringer Mengen kationischer Biopolymere zu nichtionischen Tensiden die negativen Torsions-, Feuchteretentions- und sensorischen Eigenschaften so stark verbessern, daß eine geschmeidigmachende und damit Repairwirkung eintritt.

### Fettalkoholpolyglycolether

Als nichtionische Tenside kommen in erster Linie Fettalkoholpolyglycolether der Formel (I) in Betracht,

**R**^{**1**}**O(CH**_{**2**}**CH**_{**2**}**O)**_{**n**}**H (I)**

in der R¹ für einen linearen oder verzweigten Alkyl- und/oder Alkenylrest mit 6 bis 22 Kohlenstoffatomen und n für Zahlen von 1 bis 20 steht. Typische Beispiele sind die Anlagerungsprodukte von durchschnittlich 1 bis 20 und vorzugsweise 2 bis 10 Mol Ethylenoxid an jeweils 1 Mol eines Fettalkohols wie beispielsweise Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol und Erucylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind Anlagerungsprodukte von durchschnittlich 2 bis 7 Mol Ethylenoxid an technische Fettalkohole mit 12 bis 18 Kohlenstoffatomen wie beispielsweise Kokos-, Palm-, Palmkern- oder Talgfettalkohol, die sowohl eine konventionelle als auch eine eingeengte Homologenverteilung aufweisen können. Der Anteil der Fettalkoholpolyglycolether an den erfindungsgemäßen Mitteln kann 1 bis 10 und vorzugsweise 2 bis 8 Gew.-% betragen.

### Alkyl- und/oder Alkenyloligoglykoside

Als weitere nichtionische Tenside kommen Alkyl- und/oder Alkenyloligoglykoside in Frage, die der Formel (III) folgen,

**R**^{**2**}**O-[G]**_{**p**} **(II)**

in der R² für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/ oder Alkenyloligoglykoside sind somit Alkyl- und/oder Alkenyloligoglucoside.

Die Indexzahl p in der allgemeinen Formel (II) gibt den Oligomerisierungsgrad (DP-Grad), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R² kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese anfallen. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3).

Der Alkyl- bzw. Alkenylrest R² kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

Der Anteil der Alkyl- und/oder Alkenyoligoglykoside an den erfindungsgemäßen Mitteln kann 1 bis 10 und vorzugsweise 2 bis 8 Gew.-% betragen.

### Fettsäure-N-alkylpolyhydroxyalkylamide

Als weitere Gruppe nichtionischer Tenside kommen Fettsäure-N-alkylpolyhydroxyalkylamide der Formel (III) in Betracht, 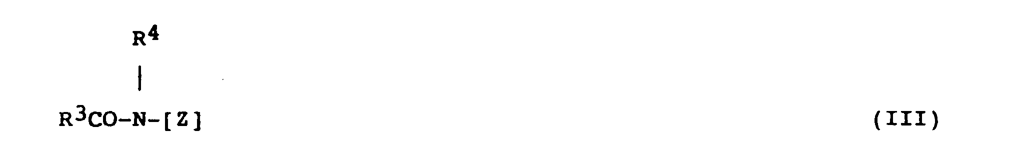 in der R³CO für einen aliphatischen Acylrest mit 6 bis 22 Kohlenstoffatomen, R⁴ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1985424, US 2016962** und **US 2703798** sowie die Internationale Patentanmeldung WO 92/06984 verwiesen. Eine Übersicht zu diesem Thema von H.Kelkenberg findet sich in **Tens. Surf.Det. 25, 8 (1988)**.

Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher **Fettsäure-N-alkylglucamide** dar, wie sie durch die Formel (IV) wiedergegeben werden:

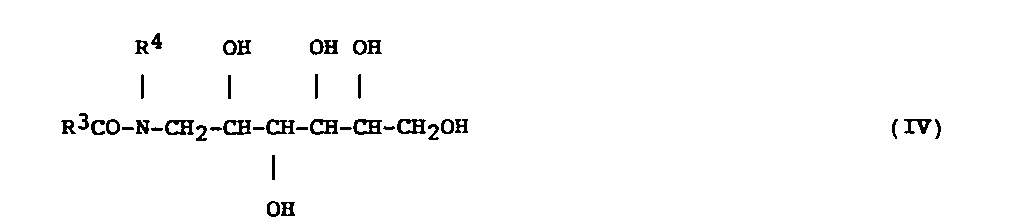

Vorzugsweise werden als Fettsäure-N-alkylpolyhydroxyalkylamide Glucamide der Formel **(IV)** eingesetzt, in der R⁴ für Wasserstoff oder eine Alkylgruppe steht und R³CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel **(IV)**, die durch reduktive Aminierung von Glucose mit Methylamin und anschließende Acylierung mit Laurinsäure oder C_{12/14}-Kokosfettsäure bzw. einem entsprechenden Derivat erhalten werden. Weiterhin können sich die Polyhydroxyalkylamide auch von Maltose und Palatinose ableiten.

Der Anteil der Fettsäure-N-alkylpolyhydroxyalkylamide an den erfindungsgemäßen Mitteln kann 1 bis 10 und vorzugsweise 2 bis 8 Gew.-% betragen.

### Kationische Biopolymere

Unter dem Begriff kationische Biopolymere sind Hydrokolloide wie z.B. Chitosan oder Hydroxypropylchitosan zu verstehen. Chemisch betrachtet handelt es sich um partiell deacetylierte Chitine unterschiedlichen Molekulargewichtes, die den - idealisierten - Monomerbaustein (V) enthalten:

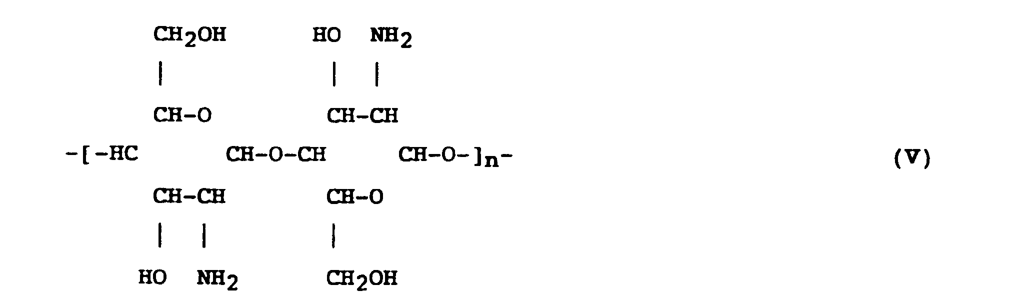

Die positiv geladenen Biopolymeren können mit entgegengesetzt geladenen Oberflächen in Wechselwirkung treten und werden daher in kosmetischen Haar- und Körperpflegemitteln eingesetzt. Übersichten zu diesem Thema sind beispielsweise von B.Gesslein et al. in **HAPPI 27, 57 (1990)**, O.Skaugrud in **Drug Cosm. Ind. 148, 24 (1991)** und E.Onsoyen et al. in **Seifen-Öle-Fette- Wachse 117, 633 (1991)** erschienen.

Zur Herstellung der Chitosane geht man von Chitin, vorzugsweise den Schalenresten von Krustentieren aus, die als billige Rohstoffe in großen Mengen zur Verfügung stehen. Das Chitin wird dabei üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Entsprechende Verfahren zur Herstellung von - mikrokristallinem - Chitosan sind beispielsweise in der **WO 91/05808** (Firextra Oy) und der **EP-B1 0382150** (Hoechst) beschrieben.

Kationische Biopolymere, die im Sinne der vorliegenden Erfindung besonders bevorzugt sind, werden erhalten, indem man
(a) frische Krustentierschalen mit verdünnter wäßriger Mineralsäure behandelt,
(b) das resultierende demineralisierte erste Zwischenprodukt mit wäßriger Alkalihydroxidlösung behandelt,
(c) das resultierende geringfügig deproteinierte zweite Zwischenprodukt erneut mit verdünnter wäßriger Mineralsäure behandelt, und
(d) das resultierende decalcifizierte dritte Zwischenprodukt schließlich mit konzentrierter wäßriger Alkalilauge behandelt und dabei bis zu einem Gehalt von 0,05 bis 0,5 und insbesondere 0,15 bis 0.25 Mol Acetamid pro Mol Monomereinheit deacetyliert.

Die auf diese Weise erhältlichen Stoffe lassen sich infolge ihres sehr geringen Aschegehaltes gegebenenfalls nach einer Temperatur/Drucknachbehandlung derart einstellen, daß sie beispielsweise in glycolsaurer Lösung eine niedrige oder hohe Viskosität aufweisen.

Die kationischen Biopolymere können in den erfindungsgemäßen Mittel in Mengen von 0,01 bis 0,25 und vorzugsweise 0,05 bis 0,1 Gew.-% - bezogen auf die Mittel - enthalten sein.

### Gewerbliche Anwendbarkeit

Die erfindungsgemäßen Hautpflegemittel können in untergeordneten Mengen weitere, mit den anderen Inhaltsstoffen kompatible **Tenside** enthalten. Typische Beispiele sind Fettalkoholpolyglycolethersulfate, Monoglyceridsulfate, Ethercarbonsäuren, Mono- und/oder Dialkylsulfosuccinate, Fettsäureisethionate, Fettsäuridesarcosinate, Fettsäuretauride, Alkylamidobetaine und/oder Proteinhydrolysate bzw. deren Kondensate mit Fettsäuren auf tierischer oder vorzugsweise pflanzlicher Basis.

Ferner können Mittel, wie beispielsweise Cremes und Lotionen einen Gehalt an Ölkörpern, Emulgatoren, Fetten und Wachsen, Stabilisatoren sowie Überfettungsmitteln, Verdickungsmitteln, biogenen Wirkstoffen, Filmbildnern, Konservierungsmitteln, Farb- und Duftstoffen aufweisen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C6-C20-Fettalkoholen, Ester von verzweigten C₆-C₁₃-Carbonsäuren mit linearen C₁₆-C₁₈-Fettalkoholen, Ester von linearen C₁₀-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimerdiol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate und/oder Dialkylether in Betracht.

Als **Emulgatoren** kommen sowohl bekannte W/O- als auch O/W-Emulgatoren wie beispielsweise gehärtetes und ethoxyliertes Ricinusöl, Polyglycerinfettsäureester oder Polyglycerinpolyricinoleate bzw. -12-hydroxystearate in Frage.

Typische Beispiele für Fette sind Glyceride, als **Wachse** kommen u.a. Bienenwachs, Paraffinwachs oder Mikrowachse gegebenenfalls in Kombination mit hydrophilen Wachsen, z.B. Cetylstearylalkohol in Frage.

Als **Stabilisatoren** können Metallsalze von Fettsäuren wie z.B. Magnesium-, Aluminium und/oder Zinkstearat eingesetzt werden.

Als **Überfettungsmittel** können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen.

Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise Fettalkoholethoxylate mit eingeengter Homologenverteilung sowie Elektrolyte wie Kochsalz und Ammoniumchlorid.

Unter **biogenen Wirkstoffen** sind beispielsweise Pflanzenextrakte und Vitaminkomplexe zu verstehen.

Gebräuchliche **Filmbildner** sind beispielsweise Polyvinylpyrrolidon, Vinylpyrrolidon-Vinyl-acetat-Copolymerisate, Polymere der Acrylsäurereihe, quaternäre Cellulose-Derivate, Kollagen, Hyaluronsäure bzw. deren Salze und ähnliche Verbindungen.

Als **Konservierungsmittel** eignen sich beispielsweise Phenoxyethanol, Formaldehydlösung, Parabene, Pentandiol oder Sorbinsäure.

Als **Perlglanzmittel** kommen beispielsweise Glycoldistearinsäureester wie Ethylenglycoldistearat, aber auch Fettsäuremonoglycolester in Betracht.

Als **Farbstoffe** können die für kosmetische Zwecke geeigneten und zugelassenen Substanzen verwendet werden, wie sie beispielsweise in der Publikation "**Kosmetische Färbemittel" der Farbstoffkommission der Deutschen Forschungsgemeinschaft, veröffentlicht im Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% und der nicht wäßrige Anteil ("Aktivsubstanzgehalt") 20 bis 80, vorzugsweise 30 bis 70 Gew.-% - bezogen auf die Mittel - betragen. Die Herstellung der Mittel kann in an sich bekannter Weise, d.h. beispielsweise durch Heiß-, Kalt-, Heiß-Heiß/Kalt- bzw. PIT-Emulgierung erfolgen. Hierbei handelt es sich um ein rein mechanisches Verfahren, eine chemische Reaktion findet nicht statt.

Ein weiterer Gegenstand der Erfindung betrifft schließlich die Verwendung von kationischen Biopolymeren, vorzugsweise Chitosanen, zur Verbesserung der dermatologischen Eigenschaften nichtionischer Tenside, vorzugsweise von Fettalkoholpolyglycolethern, Alkyl- und/oder Alkenyloligoglykosiden und/oder Fettsäure-N-alkylpolyhydroxyalkylamiden. Die Einsatzmenge der kationischen Biopolymeren kann bezogen auf die nichtionischen Tenside dabei im Bereich von 1 bis 25, vorzugsweise 0,1 bis 10 Gew.-% liegen.

Die folgenden Beispiele sollen den Gegenstand der Erfindung näher erläutern, ohne ihn darauf einzuschränken.

### Beispiele

**Torsions- und Feuchteretentionsmessung**. Zur Bestimmung der elastischen Eigenschaften wurde Schweineepidermis jeweils nach Applikation der Vergleichsrezeptur R1 und der erfindungsgemäßen Rezeptur R2 untersucht. Die Zusammensetzung der Rezepturen sind in Tabelle 1 zusammengefaßt:

**Tabelle 1:**

| Vergleichs- und Beispielrezepturen | | |
|---|---|---|
| **Komponente** | **R1** **Gew.-%** | **R2** **Gew.-%** |
| Emulgade^{(R)} SE | 6,0 | 6,0 |
| Lanette^{(R)} O | 1,5 | 1,5 |
| Cetiol^{(R)} V | 4,0 | 4,0 |
| Cetiol^{(R)} V 868 | 8,0 | 8,0 |
| Kationisches Biopolymer | - | 0,1 |
| Wasser (demineralisiert) | ad 100 | |
| Legende:Emulgade^{(R}) SE = Emulgatormischung auf Basis Fettalkoholpolyglycolether Lanette^{(R)} O = Cetearyl Alkohol Cetiol^{(R)} V = Decyl Oleate Cetiol^{(R)} 868 = Decyl Myristate | | |

In einer Meßapparatur wurden zehn Probengefäße mit Schweineepidermis bestückt. Die Oberflächen wurden rasiert und mit destilliertem Wasser sorgfältig abgespült. Anschließend wurden vier Proben mit der Vergleichsrezeptur R1 und vier weitere mit der Beispielsrezeptur R2 überdeckt. Anschließend wurden alle zehn Proben - also auch die beiden Blindproben - in einer Klimakammer bei einer Temperatur von 30°C und einer relativen Feuchte von 30 % über einen Zeitraum von 30 min konditioniert. Danach wurde die Torsionskurve der Proben, d.h. die Winkeländerung als Funktion des Drehmomentes aufgenommen. Die Meßergebnisse (jeweils Mittelwerte der zwei Blindproben bzw. der vier Rezepturen R¹ und R2) sind in Tabelle 2 zusammengefaßt.

**Tabelle 2**

| Torsionsmessungen | | | |
|---|---|---|---|
| **Drehmoment mNm** | **Winkel** | | |
| | **Blindprobe** | **R1** | **R2** |
| 0,0 | 0,00 | 0,00 | 0,00 |
| 0,5 | 0,00 | 0,00 | 0,00 |
| 1,0 | 0,25 | 0,20 | 0,30 |
| 1,5 | 0,30 | 0,25 | 0,50 |
| 2,0 | 0,70 | 0,60 | 1,60 |
| 2,5 | 1,30 | 1,00 | 3,30 |
| 3,0 | 3,00 | 2,00 | 4,80 |
| 3,5 | 4,80 | 3,50 | 9,00 |
| 4,0 | 8,25 | 5,95 | 15,90 |
| 4,5 | 15,00 | 9,80 | 30,80 |

Die Proben, die mit der Vergleichsrezeptur R1 behandelt wurden, zeigen gegenüber der Blindprobe eine deutliche Rechtsverschiebung der Winkel/Drehmomentkurve zu höheren Drehmomenten. Bei gleichen Drehmomenten weisen die behandelten Proben also niedrigere Winkel auf. Daraus folgt, daß die Haut insgesamt härter wird und sich die Stick-Slip-Grenze deutlich erhöht. Der Kurvenverlauf entspricht dem bei tensidgeschädigter Haut. Im Mittel ergibt sich eine Absenkung der erreichten Winkeländerung von 66 % relativ zum Ausgangswert.

Im Gegensatz dazu führt die erfindungsgemäße chitosanhaltige Rezeptur bei gleichen Drehmomenten zu höheren Winkeln, also einer Linksverschiebung der Verlaufkurve. Die Haut wird demnach nachgiebiger, die Stick-Slip-Grenze wird herabgesetzt. Zudem zeigt die gravimetrisch ermittelte Wasserverlustkurve im Fall der Rezeptur R2 erniedrigte Werte. Der Mittelwert für die vergrößerten Winkel nach der Applikation der chitosanhaltigen Rezeptur beträgt 85 % relativ zum Ausgangswert.

## Patentansprüche

1. Hautpflegemittel, enthaltend
(a) 1 bis 10 Gew.-% nichtionische Tenside vom Typ der (a1) Alkyl- und/oder Alkenyloligoglykoside oder (a2) Fettsäure-N-alkylpolyhydroxyalkylamide und
(b) 0,01 bis 0,25 Gew.-% Chitosan.

2. Mittel nach Anspruch 1, **dadurch gekennzeichnet**, daß sie Alkyl- und/oder Alkenyloligoglykoside der Formel **(I)** enthalten,
**R**^{**1**}**O-[G]p (I)**
in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

3. Mittel nach Anspruch 1, **dadurch gekennzeichnet**, daß sie Fettsäure-N-alkylpolyhydroxyalkylamide der Formel **(II)** enthalten, 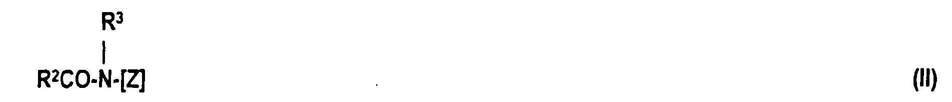 in der R²CO für einen aliphatischen Acylrest mit 6 bis 22 Kohlenstoffatomen, R³ für einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen steht.

4. Verwendung von Chitosan zur Verbesserung der dermatologischen Eigenschaften nichtionischer Tenside.

## Claims

1. Skin-care preparations containing
(a) 1 to 10% by weight of nonionic surfactants of the alkyl and/or alkenyl oligoglycoside type (a1) or the fatty acid-N-alkylpolyhydroxyalkylamide type (a2) and
(b) to 0.25% by weight of chitosan.

2. Preparations as claimed in claim 1, characterized in that they contain alkyl and/or alkenyl oligoglycosides corresponding to formula (I):
R¹O-[G]ₚ (I)
in which R¹ is an alkyl and/or alkenyl group containing 4 to 22 carbon atoms, G is a sugar unit containing 5 or 6 carbon atoms and p is an integer of 1 to 10.

3. Preparations as claimed in claim 1, characterized in that they contain fatty acid-N-alkylpolyhydroxyalkylamides corresponding to formula (II): 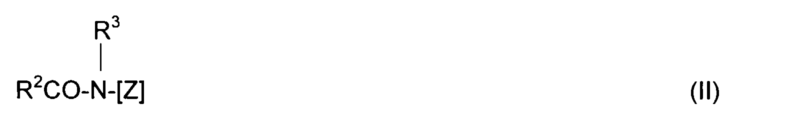 in which R²CO is an aliphatic acyl group containing 6 to 22 carbon atoms, R³ is an alkyl or hydroxyalkyl group containing 1 to 4 carbon atoms and [Z] is a linear or branched polyhydroxyalkyl group containing 3 to 12 carbon atoms.

4. The use of chitosan for improving the dermatological properties of nonionic surfactants.

## Revendications

1. Produits de soins pour la peau contenant :
a) de 1 à 10 % en poids d'agents tensioactifs non ioniques du type de,
a1) alkyl- et/ou alkényloligoglycosides ou,
a2) N-alkylpolyhydroxyalkylamides et acide gras et,
b) de 0,01 à 0,25 % en poids de chitosane.

2. Produits selon la revendication 1,
caractérisés en ce qu'
ils renferment des alkyl- et/ou alkényloligoglycosides de formule (I)
R¹O-[G]ₚ (I)
dans laquelle R¹ représente un radical alkyle et/ou alkényle ayant de 4 à 22 atomes de carbone, G représente un reste de sucre ayant 5 ou 6 atomes de carbone et p représente un nombre allant de 1 à 10.

3. Produits selon la revendication 1,
caractérisés en ce qu'
ils renferment des N-alkylpolyhydroxyalkylamides d'acide gras de formule (II), 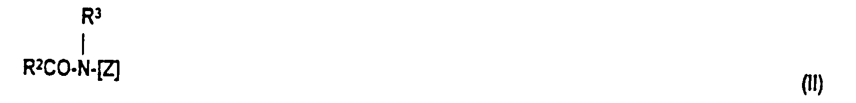 dans laquelle R²CO représente un reste acyle aliphatique ayant de 6 à 22 atomes de carbone R³, représente un reste alkyle ou hydroxyalkyle ayant de 1 à 4 atomes de carbone et [Z] représente un reste polyhydroxyalkyle linéaire ou ramifié ayant de 3 à 12 atomes de carbone.

4. Utilisation du chitosane en vue de l'amélioration des propriétés dermatologiques d'agents tensioactifs non ioniques.
